# EUROPÄISCHE PATENTSCHRIFT

(11) **EP 3 383 318 B1**
(45) Veröffentlichungstag und Bekanntmachung des Hinweises auf die Patenterteilung: **22.09.2021**
(21) Anmeldenummer: 16801779.6
(22) Anmeldetag: 28.11.2016
(51) Int. Cl.: A61F 2/16

(54) **INTRAOKULARLINSEN-KASSETTE MIT DECKEL UND INJEKTOR MIT KASSETTE**
INTRAOCULAR LENS CARTRIDGE COMPRISING A LID, AND INJECTOR COMPRISING A CARTRIDGE
CASSETTE POUR LENTILLE INTRAOCULAIRE, POURVUE D'UN COUVERCLE, ET INJECTEUR ÉQUIPÉ D'UNE TELLE CASSETTE

(30) Priorität: 03.12.2015 DE 102015224142
(43) Veröffentlichungstag der Anmeldung: 10.10.2018
(73) Patentinhaber: Carl Zeiss Meditec AG, 07745 Jena (DE)
(72) Erfinder: MÜLLER, Marco, 10437 Berlin (DE)
(74) Vertreter: Carl Zeiss AG - Patentabteilung
(86) Internationale Anmeldenummer: PCT/EP2016/078952
(87) Internationale Veröffentlichungsnummer: WO 2017/093164

(56) Entgegenhaltungen:
- WO-A1-2013/038689
- WO-A1-2013/168410
- WO-A1-2014/208507
- WO-A1-2015/076308
- WO-A2-2015/070358
- US-A1- 2010 130 985

## Beschreibung

Die Erfindung betrifft eine Kassette zur Aufnahme einer Intraokularlinse für einen Injektor sowie einen Injektor mit einer solchen Kassette.

Es ist bekannt, dass viskoelastische Materialien beziehungsweise Fluide, die auch als OVD (Ophthalmo-viscosurgical devices) bezeichnet werden, in der operativen Augenheilkunde umfänglich Anwendung finden. Bei Kataraktoperationen, bei denen eine Augenlinse zertrümmert und abgesaugt wird und dann eine Intraokularlinse eingesetzt wird, werden diese Materialien verwendet. Sie werden dabei beim operativen Eingriff auch in das Auge injiziert. Dadurch wird beispielsweise unter anderem die Stabilität der vorderen Augenkammer aufrecht erhalten. Darüber hinaus wird durch diese Materialien das Gewebe der Intraokularlinse geschützt und das Endothel der Hornhaut beziehungsweise der Kornea ebenfalls geschützt. Ferner wird dadurch in diesem operativen Eingriff an dem Auge Volumen bereitgestellt, um Gewebe präzise und zuverlässig entnehmen zu können.

Diese Fluide sind jedoch nach dem Ende des operativen Eingriffs wieder vollständig aus dem Auge zu entfernen, denn bei einem Verbleib im Auge können Komplikationen auftreten und sich insbesondere auch der Augendruck unerwünscht erhöhen.

Es ist aus der US 8 123 804 B2 eine Kassette für eine Intraokularlinse bekannt, die einen an einem wannenartigen Teil der Kassette schwenkbar angeordneten, plattenartigen Deckel aufweist, der den Volumenbereich des wannenartigen Teils verschließt. Durch den Deckel und das wannenartige Teil ist die Kassette gebildet. In dem Volumenbereich ist die Intraokularlinse aufgenommen, bevor sie ausgeschoben wird und mit einem Injektor in das Auge implantiert wird. Dieser Deckel weist ein Loch auf, welches nach außen führt und durch welches im geschlossenen Zustand der Kassette ein Gleitmittel in den Volumenbereich eingebracht werden kann.

Ein wesentlicher Nachteil ist darin zu sehen, dass durch das kleine und somit in Lochachse wenig tiefe und geradlinige Loch beim Einfüllen des Gleitmittels auch die Intraokularlinse beschädigt werden kann. Das Einführen erfolgt üblicherweise mit einer Spritze mit einer Nadel, die in das Loch eingeführt wird. Dabei kann leicht die Nadel zu weit eingeführt werden und dabei die Intraokularlinse berühren, so dass sie verkratzt und/oder verrutscht werden kann. Das Verrutschen führt dazu, dass das nachfolgende Falten der Intraokularlinse in der Injektorspitze beim Ausschiebevorgang nicht mehr so erfolgt wie es sein sollte, da die Ausgangslage vor dem Falten verändert wurde. Dies kann zu Problemen bei der Einführung ins Auge führen. Darüber hinaus ist bei diesem Stand der Technik die Kassette mit ihrem Deckel und wannenartigen Teil derart ausgebildet, dass ein Falten der Intraokularlinse erst in der an die Kassette anschließende Injektorspitze ermöglicht ist, so dass durch das Schließen des plattenartigen Deckels der Kassette keinerlei Einfluss auf die Lage und Form der Intraokularlinse in dem Volumenbereich erfolgt.

Darüber hinaus ist aus der EP 1 808 150 A1 eine Kassette für eine Intraokularlinse bekannt, welche zwei relativ zueinander schwenkbare Teile aufweist, die im geschlossenen Zustand einen Aufnahmebereich für die Intraokularlinse bilden. An einem hinteren Bereich der Teile und somit benachbart zu einem die Teile schwenkbar verbindenden Scharnier ist ein Loch zum Einfüllen eines Gleitmittels ausgebildet. Bei dieser Ausführung tritt das Problem auf, dass das Einfüllen des Gleitmittels das Falten der Linse behindert. Dadurch wird die gewünschte Faltrichtung der Linse hin zum Scharnier wesentlich beeinträchtigt und gehemmt, so dass die gefaltete Endstellung der Intraokularlinse in der Kassette nicht oder nur unzureichend erreicht wird, was wiederum nachteilig für den weiteren Ausschiebevorgang ist und zu Beschädigungen der Linse oder einer unerwünschten Ausschiebelage am vorderen Ende der Injektorspitze führt.

Aus der WO 2015/070358 A2 ist eine Vorrichtung zur Aufnahme einer Intraokularlinse bekannt. Dort ist eine Kartusche gezeigt, welche zwei direkt miteinander schwenkbar verbundene Halbschalen aufweist, an denen jeweils noch ein Flügel ausgebildet ist. Zwischen den Halbschalen erstreckt sich ein Deckel, der eine Aussparung aufweist, durch welche ein Gleitmittel in eine Kammer, in welcher die Intraokularlinse angeordnet ist, zuführbar ist. Die Zuführung kann jedoch nur im geöffneten Zustand der Flügel und somit auch der Halbschalen erfolgen. Dadurch kann der nur einseitig an nur einer Halbschale mit einem Filmscharnier befestigte Deckel angehoben werden und das Gleitmittel aus der Kammer unerwünscht entweichen. Des Weiteren ist die Aussparung in dem Deckel schwer zu treffen, so dass die Nadel einer Gleitmittel-Zuführungseinheit auch abrutschen kann. Darüber hinaus ist die Aussparung geometrisch nachteilig, da die Nadel der Gleitmittel-Zuführeinheit leicht hindurch geführt werden kann und dann die Intraokularlinse beschädigen kann.

In der WO 2015/070358 A2, WO 2015/076308 A1, WO 2013/168410 A1, US 2010/130985 A1, WO 2013/038689 A1, WO 2014/208507 A1 sind jeweils eine Kassette zur Aufnahme einer Intraokularlinse beschrieben.

Es ist eine Aufgabe der vorliegenden Erfindung, eine Kassette für eine Intraokularlinse zu schaffen, mit der eine sichere Zuführung eines Gleitmittels von außerhalb der Kassette möglich ist. Es ist ferner eine Aufgabe, einen Injektor zum Einführen einer Intraokularlinse in ein Auge zu schaffen, welcher eine derartige Kassette aufweist.

Die Aufgabe wird für die Kassette durch den Gegenstand des Patentanspruchs 1 gelöst. Vorteilhafte Weiterbildungen der Erfindung sind Gegenstand der Unteransprüche. Die Aufgabe wird für den Injektor durch den Gegenstand des Patentanspruchs 9 gelöst.

Bezüglich der nachfolgend verwendeten Begriffe "Erfindung" oder "Ausführungsform" und/oder Merkmalen, die als bevorzugt oder optional bezeichnet sind, wird darauf hingewiesen, dass sie derart zu interpretieren sind, dass der Schutz sich nur auf die Erfindung bezieht, welche mit den Patentansprüchen beansprucht ist.

Eine erfindungsgemäße Kassette ist zur Aufnahme einer Intraokularlinse ausgebildet. Sie ist daher eine Intraokularlinsen-Aufnahmekassette. Die Kassette ist für einen Injektor zum Einführen der Intraokularlinse in ein Auge ausgebildet. Die Kassette umfasst zwei um eine Längsachse der Kassette relativ zueinander schwenkbare Kassettenteile. Ein Kassettenteil umfasst ein rinnenartiges und somit auch bauchiges Basiselement und einen daran anschließenden plattenartigen Flügel. Ein weiteres Kassettenteil umfasst ebenfalls ein rinnenartiges und somit bauchiges Basiselement und einen daran anschließenden plattenartigen Flügel. Die Kassettenteile sind jeweils insbesondere einstückig ausgebildet. Durch die Basiselemente ist im geschlossenen Zustand der Kassette zumindest ein Teil einer Aufnahmekammer für die Intraokularlinse gebildet. Dies bedeutet, dass im geschlossenen Zustand der Kassette durch die beiden Basiselemente mindestens ein Teil einer, insbesondere röhrenförmigen, Aufnahmekammer für die Intraokularlinse und somit eine Intraokularlinsen-Aufnahmekammer gebildet ist. Die Kassette umfasst darüber hinaus eine Gleitmittel-Zufiihreinrichtung, mit welcher ein Gleitmittel für die Intraokularlinse von außerhalb der Kassette in die Aufnahmekammer zuführbar ist.

Ein wesentlicher Gedanke der Erfindung ist darin zu sehen, dass zumindest ein Kassettenteil mit einem Deckel zum Abdecken der Aufnahmekammer gelenkig verbunden ist und ein Einfüllloch der Gleitmittel-Zuführeinrichtung in diesem Deckel ausgebildet ist. Mit dem Deckel, der zur Abdeckung der Aufnahmekammer angeordnet ist, ist zusätzlich zu den beiden Kassettenteilen zumindest ein drittes Teil der Kassette bereitgestellt. Mit dem Deckel ist es möglich, die Aufnahmekammer auch im noch nicht geschlossenen Zustand der Kassette von oben abzudecken. Dies bedeutet, dass auch dann, wenn die beiden Kassettenteile durch eine Schwenkbewegung noch nicht vollständig in ihrer geschlossenen Endlage angeordnet sind, dennoch die Aufnahmekammer durch den Deckel abgedeckt ist. Der Deckel ist derart ausgebildet, dass sich Gleitmittel von außerhalb der Kassette in die Aufnahmekammer zuführen lässt. Dies erfolgt mittels des Einfüllloches, welches ein Durchgangsloch ist. Durch eine derartige Ausgestaltung sind mehrere Vorteile erreicht. Zum einen ist eine in der Aufnahmekammer angeordnete Intraokularlinse auch im geöffneten Zustand der Kassette durch den Deckel abgedeckt und kann somit nicht herausfallen oder bei einer Berührung mit anderen Gegenständen beschädigt werden. Darüber hinaus ist es durch diese Ausgestaltung mit dem an dem Deckel ausgebildeten Einfüllloch auch möglich, dass bereits im geöffneten Zustand der Kassette eine Befüllung der Aufnahmekammer mit dem Gleitmittel einfach erfolgen kann. Durch diese ganz spezifische Lage des Deckels und des Einfülllochs ist es in besonders vorteilhafter Weise möglich, dass im noch geöffneten Zustand der Kassette und im dann noch nicht vorgefalteten Zustand der Intraokularlinse nach dem Zuführen von Gleitmittel in das Einfüllloch ein Anpressen beziehungsweise Stabilisieren der Ausgangslage der Intraokularlinse erreicht ist.

Durch diese Ausgestaltung ist es möglich, dass ein Gleitmittel nicht von der seitlichen vorderen oder hinteren Öffnung der Aufnahmekammer eingefüllt werden muss. Stattdessen ist mittels der erfindungsgemäßen Kassette eine Befüllung der Aufnahmekammer über ein in axialer Richtung zwischen der vorderen und hinteren Öffnung der Aufnahmekammer liegendes Einfüllloch durchführbar.

Ein weiterer wesentlicher Gedanke der Erfindung ist es, dass an einer der Aufnahmekammer abgewandten Außenseite des Deckels ein Stutzen vorsteht, der einen Einlauf aufweist, der in das Einfüllloch mündet. Der Stutzen ist somit ein hohlkörperartiger Aufsatz der sich von der Außenseite des Deckels fort erstreckt. Dies ist eine sehr vorteilhafte Ausführung, da somit das Einfüllloch für einen Bediener einfach erkennbar ist. Mit dem Stutzen ist es darüber hinaus ermöglicht, dass ein Hilfswerkzeug zum Einfüllen des Gleitmittels in die Aufnahmekammer einfach und auch sicher sowie zielgerichtet zum Einfüllloch gelangen kann. Darüber hinaus ist durch den Stutzen auch erreicht, dass das daran anliegende Hilfswerkzeug, insbesondere mit einer Injektionsnadel, beim Einfüllen des Gleitmittels von dem Hilfswerkzeug in die Aufnahmekammer in der Position stabilisiert ist. Ein Abrutschen und somit ein unerwünschtes Verteilen des Gleitmittels außerhalb der Aufnahmekammer ist dadurch verhindert. Der Stutzen ist dadurch auch ein Führungsteil bzw. Stabilisierungsteil für das Hilfswerkzeug beim Einfüllen des Gleitmittels in die Aufnahmekammer, da sich das Hilfswerkzeug dann in den Stutzen hinein erstreckt.

Insbesondere weist der Einlauf im Stutzen an seiner schmalsten Stelle eine Breite oder einen Innendurchmesser auf, der kleiner ist als ein Außendurchmesser einer Injektionsnadel eines Hilfswerkzeugs, mit welchem Gleitmittel eingefüllt wird. Insbesondere weist das Einfüllloch zusätzlich oder anstatt dazu an seiner schmalsten Stelle einen Innendurchmesser auf, der kleiner ist als ein Außendurchmesser einer Injektionsnadel eines Hilfswerkzeugs, mit welchem Gleitmittel eingefüllt wird. Durch eine derartige Ausgestaltung kann es vermieden werden, dass eine Injektionsnadel eines Hilfswerkzeugs, wenn sie mit dem Stutzen gekoppelt wird, nicht unerwünscht direkt bis zur Aufnahmekammer durchgestochen werden kann, sondern maximal bis zum Boden des Einlaufs eintaucht. Ein unerwünschtes Beschädigen der Intraokularlinse durch eine in die Aufnahmekammer eindringende Injektionsnadel des Hilfswerkzeugs ist dadurch vermieden. Ebenso kann es dadurch vermieden werden, dass durch eine Injektionsnadel eine Intraokularlinse in der Aufnahmekammer in ihrer Grundposition verschoben wird.

Bevorzugt ist die kleinste Querschnittsbreite des Einlaufs größer als die größte Querschnittsbreite des Einfüllloches. Damit lässt sich erreichen, dass eine Injektionsnadel zuverlässig in den Einlauf eingeführt werden kann, jedoch oberhalb des Einfüllloches verbleibt und nicht durch das Einfüllloch durchgestochen werden kann. Eine Beschädigung einer in die Kassette eingesetzte Intraokularlinse lässt sich somit verhindern.

Vorzugsweise ist vorgesehen, dass der Stutzen einen trichterförmig ausgebildeten Einlauf aufweist, dessen verjüngtes und distales Ende in das Einfüllloch mündet. Diese vorteilhafte Ausführung ermöglicht das zielgerichtete Zuführen des Hilfswerkzeugs zum Einfüllloch besonders vorteilhaft und die stabilisierende Position des Hilfswerkzeugs beim Einfüllvorgang des Gleitmittels ist nochmals verbessert.

Insbesondere ist der Stutzen zylinderförmig ausgebildet. Dadurch lässt sich auch eine symmetrische Geometrie schaffen, die insbesondere bei einer Ausgestaltung mit einem trichterförmigen Einlauf vorteilhaft wirkt, da dann der trichterförmige Einlauf rotationssymmetrisch um die Lochachse des Einfülllochs ausgebildet werden kann. Die Injektionsnadel des Hilfswerkzeugs wird dann auch bei einer außermittigen Einführung in den Einlauf des Stutzens durch die glatten, geneigten Wände der Trichterform nach dem Prinzip der Selbsthilfe mittig ausgerichtet.

Vorzugsweise ist vorgesehen, dass ein Kassettenteil, insbesondere in seinem Flügel, eine Aussparung aufweist, durch welche der Stutzen und somit auch das Einfüllloch im geschlossenen Zustand der Kassette von außen zugänglich ist. Dies ist eine besonders vorteilhafte Ausführung, da somit wahlweise auch im geschlossenen Zustand der Kassette das Einfüllen von Gleitmittel in die Aufnahmekammer problemlos ermöglicht ist. Der Deckel legt sich dabei derart zwischen die Flügel, dass das Einfüllloch nach außen hin durch die Aussparung zugänglich ist. Ein unerwünschter Austritt von Gleitmittel aus der Aufnahmekammer ist dadurch im besonderen Maße verhindert.

Vorzugsweise erstreckt sich der Stutzen im geschlossenen Zustand der Kassette in die Aussparung in dem Kassettenteil hinein. Das zielsichere Kontaktieren mit einem Hilfswerkzeug, welches zum Zuführen des Gleitmittels vorgesehen ist, ist dadurch verbessert.

Vorzugsweise ist vorgesehen, dass eine der Aufnahmekammer abgewandte Außenseite des Deckels im geschlossenen Zustand der Kassette an einer Innenseite eines Flügels eines Kassettenteils, insbesondere eines Kassettenteils mit einer Aussparung zur Zugänglichkeit des Einfülllochs von außerhalb der Kassette, anliegt. Dadurch wird eine stabilisierende Position des Deckels zu einem Kassettenteil erreicht, über das sich das Einfüllloch nicht unerwünscht verschieben oder bewegen kann oder dann, wenn ein Hilfswerkzeug damit mechanisch kontaktiert wird, dennoch die Position des Einfülllochs zuverlässig beibehalten ist. Auch dadurch kann dann ein Abrutschen des Hilfswerkzeugs vom Einfüllloch verhindert werden.

Vorzugsweise ist vorgesehen, dass in einem Kassettenteil und/oder in dem Deckel ein rinnenförmiger bzw. rinnenartiger Zuführkanal der Gleitmittel-Zuführeinrichtung ausgebildet ist, der in die Aufnahmekammer mündet und im geschlossenen Zustand der Kassette mit dem Einfüllloch zur Einleitung von Gleitmittel vom Einfüllloch zur Aufnahmekammer korrespondiert beziehungsweise zusammenwirkt. Dies ist vorteilhaft, da bei spezifischen Positionen des Einfülllochs und spezifischer Lage des Deckels das Gleitmittel dann über den rinnenartigen Zuführkanal dennoch in die Aufnahmekammer gelangt und durch die Orientierung und das Einmünden des rinnenartigen Zuführkanals in die Aufnahmekammer auch eine örtlich spezifische Einleitposition des Gleitmittels, insbesondere unabhängig von der Lage des Stutzens und des Einfülllochs, in die Aufnahmekammer vorgegeben werden kann.

Insbesondere ist ein rinnenartiger Zuführkanal ein in Umlaufrichtung um eine Längsachse des Zuführkanals betrachtet nicht vollständig geschlossener Kanal. Eine Rinne ist somit in Umlaufrichtung der Kanalachse beziehungsweise Längsachse des Zuführkanals bereichsweise geöffnet. Diese ist über zumindest eine Teillänge des rinnenartigen Zuführkanals ausgebildet, vorzugsweise über seine gesamte Länge entsprechend ausgebildet.

Der rinnenartige Zuführkanal ist vorzugsweise entlang seiner Kanalachse vollständig geradlinig ausgebildet. Dadurch kann ein unerwünschter Gleitmittelstau oder eine Verstopfung des Zuführkanals vermieden werden.

Vorzugsweise mündet das Einfüllloch direkt in den rinnenartigen Zuführkanal. Insbesondere ist vorgesehen, dass die Lochachse des Einfülllochs in einem Winkel zwischen 60° und 120°, insbesondere 80° und 100 °, zur Längsachse bzw. Kanalachse des rinnenartigen Zuführkanals orientiert ist. Auch durch eine derartige Ausgestaltung kann es vermieden werden, dass eine Injektionsnadel eines Hilfswerkzeugs, wenn sie mit dem Einfüllloch gekoppelt wird, nicht unerwünscht direkt bis zur Aufnahmekammer durchgestochen werden kann, sondern maximal bis zum Boden des Einfülllochs eintaucht und durch diese winklige Anordnung zwischen dem Einfüllloch und dem rinnenartigen Zuführkanal dann gestoppt wird. Ein unerwünschtes Beschädigen der Intraokularlinse durch eine eindringende Injektionsnadel des Hilfswerkzeugs ist dadurch vermieden. Ebenso kann es dadurch vermieden werden, dass durch eine Injektionsnadel eine Intraokularlinse in der Aufnahmekammer in ihrer Grundposition verschoben wird.

Vorzugsweise ist im geschlossenen Zustand der Kassette der Zuführkanal in einer Ebene senkrecht zu einer Lochachse des Einfülllochs verlaufend angeordnet und das Einfüllloch mündet an dem Zuführkanal.

Vorzugsweise ist der rinnenartige Zuführkanal in dem Flügel dieses Kassettenteils ausgebildet. Im teilweise geschlossenen oder vollständig geschlossenen Zustand der Kassette kann das Gleitmittel somit vom Stutzen in das Einfüllloch und von dort zum rinnenartigen Zuführkanal in den Aufnahmeraum gelangen, wobei das Gleitmittel auf einer Oberseite der im Aufnahmeraum platzierten Intraokularlinse auftrifft. Das Gleitmittel übt somit eine Kraft auf die Oberseite der Intraokularlinse aus, wodurch das Falten der Intraokularlinse in eine C-Form unterstützt wird.

Es kann auch vorgesehen sein, dass der Zuführkanal an einer der Aufnahmekammer zugewandten Innenseite des Deckels ausgebildet ist.

Die Vorfaltung der Intraokularlinse in der Kassette beim Schließen der Kassette erfolgt insbesondere derart, dass die Intraokularlinse in Richtung des jeweiligen Filmscharniers der Basiselemente gebogen wird und sich somit an die jeweilige Innenseite der Basiselemente anlegt. In einem Querschnitt senkrecht zur Längsachse der Kassette ist die Intraokularlinse dann im vorgefalteten Zustand im Wesentlichen C-förmig, wobei sich die Öffnung dieser C-Form zum Deckel und somit auch zum Einfüllloch hin orientiert.

Vorzugsweise ist vorgesehen, dass eine Längsachse bzw. Kanalachse des rinnenartigen Zuführkanals geradlinig ausgebildet ist und in einem Winkel zwischen 60° und 120°, insbesondere zwischen 80° und 100° zur Längsachse der Kassette orientiert ist. Damit kann eine bezüglich der Lage des Einfülllochs örtlich individuelle Einmündestelle in die Aufnahmekammer erreicht werden und dann ein besonders gleichförmiges Verteilen des Gleitmittels in der Aufnahmekammer erfolgen. Auch ist dadurch die Länge des Zuführkanals minimiert.

Insbesondere erstreckt sich der rinnenartige Zuführkanal nur bereichsweise über die senkrecht zur Längsachse der Kassette bemessene Erstreckung des Flügels oder des Deckels. Er ist somit ein einseitig geschlossener rinnenartiger Zuführkanal und bildet somit einen Sackkanal. Ein Auslaufen des Gleitmittels in eine Richtung, welche der Aufnahmekammer abgewandt ist, ist dadurch verhindert.

Vorzugsweise ist das Einfüllloch in Richtung der Längsachse der Kassette betrachtet in einem mittleren Längendrittel des Deckels ausgebildet. Durch eine derartige mittlere Positionierung des Einfülllochs kann wiederum eine sehr gleichmäßige Einbringung des Gleitmittels in die Aufnahmekammer und in alle Richtungen eine sehr gleichmäßige Verteilung des Gleitmittels erfolgen.

Vorzugsweise ist die Kassette mit den Kassettenteilen einstückig ausgebildet, insbesondere aus Kunststoff. Die beiden Kassettenteile sind vorzugsweise direkt miteinander verbunden. Es kann dazu vorgesehen sein, dass dazu ein Filmscharnier ausgebildet ist. Dieses Filmscharnier ist insbesondere an Enden der Basiselemente ausgebildet, die den jeweiligen Flügeln der Kassettenteile abgewandt sind. Bei dieser Ausführung sind die Basiselemente daher direkt miteinander verbunden und mittels des Filmscharniers zueinander verschwenkbar.

Bei einer weiteren bevorzugten Ausführung sind die Basiselemente nicht direkt miteinander verbunden, sondern über ein Sockelelement indirekt miteinander verbunden. Es ist bei dieser Ausführung bevorzugt das Basiselement eines Kassettenteils mit einem Filmscharnier mit einem die Aufnahmekammer begrenzenden Sockelelement verbunden, und das Basiselement des weiteren Kassettenteils mit einem weiteren Filmscharnier mit dem Sockelelement verbunden. Dadurch lassen sich Alternativen einer Aufnahmekammer ausbilden.

Das Basiselement eines Kassettenteils kann auch mit mehreren Sockelelementen mit dem Basiselement des anderen Kassettenteils schwenkbar verbunden sein.

Bei den Ausführungen mit zumindest einem Sockelelement bilden die Innenseiten der Basiselemente in ihrer Gesamtheit einen Teil einer Wandung für den Aufnahmeraum bzw. die Aufnahmekammer der Intraokularlinse, und eine Innenseite des Sockelelements bildet einen weiteren Teil dieser Wandung. Eine solche Ausführungsform ist vorteilhaft, da sich somit die Querschnittgeometrie der Aufnahmekammer gut an den erforderlichen Platz für eine vorgefaltete Intraokularlinse anpassen lässt.

Es kann auch vorgesehen sein, dass der rinnenartige Zuführkanal entlang seiner Kanalachse nicht-geradlinig ausgebildet ist. Der rinnenartige Zuführkanal kann gemäß einer Ausführungsform einfach oder mehrfach gekrümmt und damit "schlangenförmig" ausgebildet sein oder mit einem Winkelversatz entlang seiner Kanalachse versehen sein. Eine derartige Ausgestaltung erhöht den Strömungswiderstand des Zuführkanals und kann bei einem Gleitmittel mit relativ geringer Viskosität die Geschwindigkeit begrenzen, mit der Gleitmittel in die Aufnahmekammer eindringt.

Vorzugsweise ist vorgesehen, dass der Deckel als Platte, insbesondere ebene Platte, ausgebildet ist, die einteilig und insbesondere eben gestaltet ist. Durch diese Ausführung des Deckels wird eine sehr flachbauende Komponente bereitgestellt, die im geöffneten Zustand der Kassette die Aufnahmekammer im Volumen nicht beeinträchtigt, andererseits im geschlossenen Zustand der Kassette einen Aufbau der Kassette unwesentlich vergrößert.

Insbesondere ist der Deckel im geschlossenen Zustand der Kassette als einlagige Zwischenplatte beziehungsweise Zwischenlage zwischen den Flügeln der Kassettenteile angeordnet. Der Deckel ist dabei mit einer Oberfläche an einer Innenseite eines Flügels anliegend angeordnet und mit der gegenüberliegenden anderen Oberfläche an einer Innenseite des Flügels des anderen Kassettenteils vollflächig anliegend.

Der Deckel ist vorzugsweise nur an einer Längsseite - Richtung parallel zur Längsachse der Kassette - mit einem Filmscharnier mit einem Kassettenteil schwenkbar zu diesem Kassettenteil verbunden. An der gegenüberliegenden Längsseite, welche ein distales Ende des Deckels bildet, ist der Deckel nur auf dem anderen Kassettenteil verschiebbar aufliegend angeordnet. Insbesondere liegt der Deckel dort auf einer Innenseite eines Flügels dieses Kassettenteils auf. Der Deckel kann dann beim Bewegen zumindest eines Kassettenteils auf dieser Innenseite entlang gleiten. Vorzugsweise weist der Deckel an dieser nicht fest angebundenen Längsseite eine Wölbung auf, welche von der Innenseite des Flügels absteht, so dass ein Verklemmen oder Spreizen bei der Relativbewegung zu diesem anderen Kassettenteil verhindert ist.

Bei einer alternativen Ausführung kann vorgesehen sein, dass der Deckel an gegenüberliegenden Längsseiten über jeweils eine Gelenkverbindung, insbesondere jeweils ein Filmscharnier, mit den Kassettenteilen gelenkig verbunden ist. Insbesondere ist der Deckel hier zumindest zweiteilig ausgebildet. Insbesondere weist der Deckel ein erstes Plattenteil auf, an dem der Stutzen ausgebildet ist, und weist zumindest ein zweites Plattenteil auf. Die Plattenteile sind mit einem Filmscharnier schwenkbar direkt miteinander verbunden, und das erste Plattenteil ist mit einem Filmscharnier mit dem ersten Kassettenteil, und das zweite Plattenteil ist mit einem Filmscharnier mit dem zweiten Kassettenteil verbunden. Der Deckel weist somit bei dieser Ausführung bevorzugt zumindest zwei gelenkig miteinander verbundene Plattenteile auf. Dadurch ergibt sich eine ziehharmonikaartige Faltung des Deckels beim Schließen der Kassette.

Insbesondere sind die beiden Plattenteile gleich groß, so dass sie im geschlossenen Zustand der Kassette deckungsgleich aufeinander liegen.

Bevorzugt sind die Basiselemente der Kassettenteile an den den Flügeln abgewandten Enden mit einem Filmscharnier direkt miteinander schwenkbar verbunden.

Der Stutzen bei diesen Ausführungen eines mehrteiligen Deckels ist an einer Außenseite nur eines Plattenteils ausgebildet. Abhängig von der Größe und Anordnung der Plattenteile sowie der Position der gelenkigen Verbindung zwischen den Plattenteilen sowie der Position des Stutzens an einem Plattenteil kann es dann erforderlich sein, dass in dem Plattenteil, an dem der Stutzen nicht angeordnet ist, ein Durchgangsloch als Einfüllloch angeordnet ist. Dadurch kann dann das Gleitmittel durch den Stutzen und die zumindest beiden Plattenteile in die Aufnahmekammer gelangen. Durch diese Ausgestaltung wird auch einerseits im geöffneten Zustand der Kassette der maximale Öffnungswinkel begrenzt, und die beiden Kassettenteile können maximal über die Breite des Deckels voneinander entfernt werden. Dadurch kann auch verhindert werden, dass die Kassette unerwünscht weit geöffnet ist und das Handhaben durch eine Bedienperson erschwert wäre. Darüber hinaus ist es bei dieser Ausführung auch erreicht, dass beim Einfüllen von Gleitmittel, insbesondere im geöffneten Zustand der Kassette, sich der Deckel nicht von einem Kassettenteil abhebt.

Vorzugsweise ist vorgesehen, dass eine feste Verbindung des Deckels mit einem Kassettenteil an einem innenseitigen Übergang zwischen dem Basiselement und dem Flügel ausgebildet ist. Die oben genannten Vorteile werden dadurch nochmals verbessert.

In vorteilhafter Weise ist vorgesehen, dass die Kassette einstückig mit einer Injektorspitze des Injektors zum Implantieren einer Intraokularlinse in ein Auge ausgebildet ist. Die Injektorspitze stellt dabei das Bauteil des Injektors dar, welches mit dem vorderen Ende in das Auge eingeführt wird, um dann die Intraokularlinse mittels des Injektors über die Injektorspitze einzuführen. Insbesondere ist vorgesehen, dass eines der beiden Kassettenteile positionsfixiert mit der Injektorspitze verbunden ist und somit keine Relativbewegung zwischen der Injektorspitze und diesem Kassettenteil ermöglicht ist. Das dann noch verbleibende weitere Kassettenteil ist dann relativ zu der Injektorspitze und zu dem anderen Kassettenteil verschwenkbar. Dieses verschwenkbare Kassettenteil ist insbesondere dasjenige, an dem der Deckel gelenkig angebunden ist, wenn der Deckel nur einseitig mit einem Kassettenteil örtlich fest aber relativ zueinander beweglich verbunden ist.

Insbesondere ist vorgesehen, dass das relativ zu dem anderen Kassettenteil und zur Injektorspitze verschwenkbare Kassettenteil mit dem Einfüllloch ausgebildet ist.

Eine erfindungsgemäße Kassette gemäß einem weiteren unabhängigen Aspekt der Erfindung ist zur Aufnahme einer Intraokularlinse ausgebildet. Sie ist daher eine Intraokularlinsen-Aufhahmekassette. Die Kassette ist für einen Injektor zum Einführen der Intraokularlinse in ein Auge ausgebildet. Die Kassette umfasst zwei um eine Längsachse der Kassette relativ zueinander schwenkbare Kassettenteile. Ein Kassettenteil umfasst ein rinnenartiges und somit auch bauchiges Basiselement und einen daran anschließenden plattenartigen Flügel. Ein weiteres Kassettenteil umfasst ebenfalls ein rinnenartiges und somit bauchiges Basiselement und einen daran anschließenden plattenartigen Flügel. Die Kassettenteile sind jeweils insbesondere einstückig ausgebildet. Durch die Basiselemente ist im geschlossenen Zustand der Kassette zumindest ein Teil einer Aufnahmekammer für die Intraokularlinse gebildet. Dies bedeutet, dass im geschlossenen Zustand der Kassette durch die beiden Basiselemente mindestens ein Teil einer, insbesondere röhrenförmigen, Aufnahmekammer für die Intraokularlinse und somit eine Intraokularlinsen-Aufnahmekammer gebildet ist. Die Kassette umfasst darüber hinaus eine Gleitmittel-Zuführeinrichtung, mit welcher ein Gleitmittel für die Intraokularlinse von außerhalb der Kassette in die Aufnahmekammer zuführbar ist.

Ein wesentlicher Gedanke der Erfindung des weiteren unabhängigen Aspekts ist darin zu sehen, dass zumindest ein Kassettenteil mit einem Deckel zum Abdecken der Aufnahmekammer gelenkig verbunden ist und ein Einfüllloch der Gleitmittel-Zuführeinrichtung in diesem Deckel der Kassette ausgebildet ist. Mit dem Deckel, der zur Abdeckung der Aufnahmekammer angeordnet ist, ist zusätzlich zu den beiden Kassettenteilen zumindest ein drittes Teil der Kassette bereitgestellt. Mit dem Deckel ist es möglich, die Aufnahmekammer auch im noch nicht geschlossenen Zustand der Kassette von oben abzudecken. Dies bedeutet, dass auch dann, wenn die beiden Kassettenteile durch eine Schwenkbewegung noch nicht vollständig in ihrer geschlossenen Endlage angeordnet sind, dennoch die Aufnahmekammer durch den Deckel abgedeckt ist. Der Deckel ist derart ausgebildet, dass sich Gleitmittel von außerhalb der Kassette in die Aufnahmekammer zuführen lässt. Dies erfolgt mittels des Einfüllloches, welches ein Durchgangsloch ist. Durch eine derartige Ausgestaltung sind mehrere Vorteile erreicht. Zum einen ist eine in der Aufnahmekammer angeordnete Intraokularlinse auch im geöffneten Zustand der Kassette durch den Deckel abgedeckt und kann somit nicht herausfallen oder bei einer Berührung mit anderen Gegenständen beschädigt werden. Darüber hinaus ist es durch diese Ausgestaltung mit dem an dem Deckel ausgebildeten Einfüllloch auch möglich, dass bereits im geöffneten Zustand der Kassette eine Befüllung der Aufnahmekammer mit dem Gleitmittel einfach erfolgen kann. Durch diese ganz spezifische Lage des Deckels und des Einfülllochs ist es in besonders vorteilhafter Weise möglich, dass im noch geöffneten Zustand der Kassette und im dann noch nicht vorgefalteten Zustand der Intraokularlinse nach dem Zuführen von Gleitmittel in das Einfüllloch ein Anpressen beziehungsweise Stabilisieren der Ausgangslage der Intraokularlinse erreicht ist.

Durch diese Ausgestaltung ist es möglich, dass ein Gleitmittel nicht von der seitlichen vorderen oder hinteren Öffnung der Aufnahmekammer eingefüllt werden muss. Stattdessen ist eine Befüllung der Aufnahmekammer über ein in axialer Richtung zwischen der vorderen und hinteren Öffnung der Aufnahmekammer liegendes Einfüllloch durchführbar.

Ein weiterer wesentlicher Gedanke der Erfindung des weiteren unabhängigen Aspekts ist darin zu sehen, dass ein Kassettenteil, insbesondere in seinem Flügel, eine Aussparung aufweist, durch welche das Einfüllloch im geschlossenen Zustand der Kassette von außen zugänglich ist. Dies ist eine vorteilhafte Ausführung, da somit wahlweise auch im geschlossenen Zustand der Kassette das Einfüllen von Gleitmittel in die Aufnahmekammer problemlos ermöglicht ist. Der Deckel legt sich dabei derart zwischen die Flügel, dass das Einfüllloch nach außen hin durch die Aussparung zugänglich ist.

Ausführungen des ersten unabhängigen erfindungsgemäßen Aspekts der Kassette sind als vorteilhafte Ausführungen des weiteren erfindungsgemäßen unabhängigen Aspekts der Kassette anzusehen.

Des Weiteren betrifft die Erfindung auch einen Injektor zum Einführen einer Intraokularlinse in ein Auge, welcher eine Kassette gemäß der oben genannten Erfindung oder einer vorteilhaften Ausgestaltung davon aufweist. Die Kassette kann als separates Teil bereitgestellt sein, welches zum Einsatz in einen Ladebereich des Injektors, insbesondere in einen Ladebereich einer Injektorröhre, ausgebildet ist. Es kann jedoch auch vorgesehen sein, dass die Kassette bereits in den Injektor integriert ist und damit einstückig verbunden ist.

Der Injektor weist vorzugsweise auch einen Kolben auf, der axial verschiebbar ist. Mittels dieses Kolbens kann dann eine axiale Ausschiebung der Intraokularlinse aus der Kassette in Richtung einer Injektorspitze des Injektors erfolgen. Insbesondere wird die in der Kassette durch den Schließvorgang der Kassette bereits vorgefaltete Intraokularlinse dann auf dem weiteren Ausschiebeweg in der Injektorspitze in ihre endgültige Faltlage gebracht und dann beim Austritt aus der Injektorspitze entsprechend gefaltet in das Auge eingeführt. Mit Angaben "oben", "unten", "vome", "hinten, "horizontal", "vertikal", "außen", "innen" etc. sind die bei bestimmungsgemäßen Gebrauch und bestimmungsgemäßem Anordnen der Kassette, insbesondere an einem Injektor zur Injektion einer Intraokularlinse in ein Auge, gegebenen Positionen und Orientierungen angegeben.

Weitere Merkmale der Erfindung ergeben sich aus den Ansprüchen, den Figuren und der Figurenbeschreibung.

Ausführungsbeispiele der Erfindung werden nachfolgend anhand schematischer Zeichnungen näher erläutert. Es zeigen:
- Fig. 1: eine perspektivische vereinfachte Darstellung eines Ausführungsbeispiels eines erfindungsgemäßen Injektors mit einem Ausführungsbeispiel einer erfindungsgemäßen Kassette;
- Fig. 2: eine perspektivische Darstellung eines Ausführungsbeispiels einer Kassette in geöffnetem Zustand;
- Fig. 3: die Kassette gemäß Fig. 2 mit einem dazu gekoppelten Hilfswerkzeug zum Einführen eines Gleitmittels in eine Aufnahmekammer;
- Fig. 4: eine andere perspektivische Darstellung der Komponenten in Fig. 3;
- Fig. 5: eine perspektivische Darstellung der Kassette gemäß Fig. 2 bis Fig. 4 im geschlossenen Zustand;
- Fig. 6: ein weiteres Ausführungsbeispiel einer Kassette im geöffneten Zustand;
- Fig. 7: eine Darstellung eines weiteren Ausführungsbeispiels einer Kassette in einer Seitenansicht im geöffneten Zustand;
- Fig. 8: die Seitenansicht gemäß Fig. 7 im geschlossenen Zustand der Kassette;
- Fig. 9: eine perspektivische Darstellung eines weiteren Ausführungsbeispiels einer Kassette im geöffneten Zustand der Kassette;
- Fig. 10: die Ansicht gemäß Fig. 9 im geschlossenen Zustand der Kassette;
- Fig. 11: eine perspektivische Darstellung eines weiteren Ausführungsbeispiels einer Kassette im geöffneten Zustand der Kassette;
- Fig. 12: eine Querschnittsansicht eines in dem Deckel der Kassette ausgebildeten ersten Stutzens;
- Fig. 13: eine Querschnittsansicht eines in dem Deckel der Kassette ausgebildeten zweiten Stutzens; und
- Fig. 14: eine Querschnittsansicht eines in dem Deckel der Kassette ausgebildeten dritten Stutzens.

In den Figuren werden gleiche oder funktionsgleiche Elemente mit den gleichen Bezugszeichen versehen.

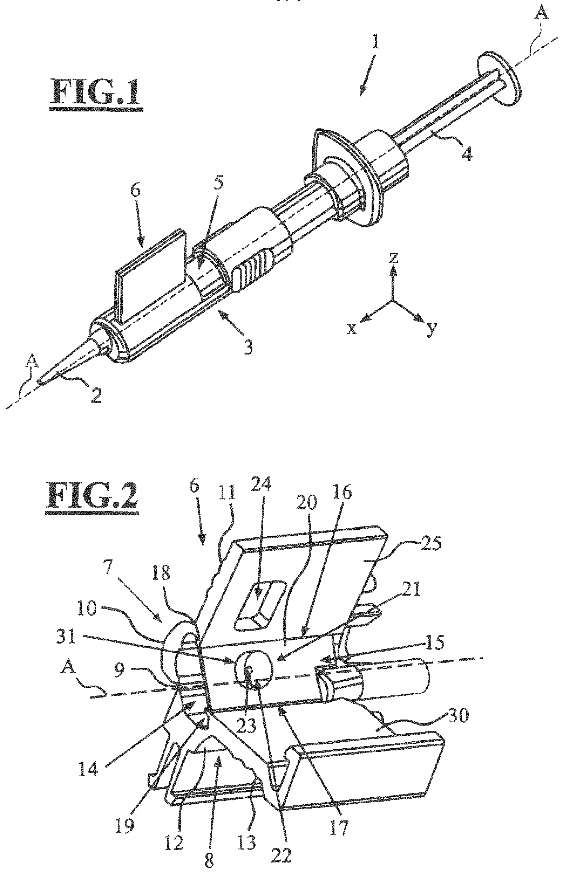

In Fig. 1 ist in einer perspektivischen Darstellung ein Injektor 1 gezeigt. Der Injektor 1 ist zum Einführen einer Intraokularlinse in ein Auge ausgebildet. Die lediglich schematisch zu verstehende Darstellung des Injektors 1 zeigt eine Injektorspitze 2, die in Richtung einer Längsachse A des Injektors 1 frontseitig angeordnet ist. Die Injektorspitze 2 wird dann bei einer Implantation bereichsweise in das Auge eingeführt und die Intraokularlinse über die Injektorspitze 2 ins Auge implantiert.

Der Injektor 1 umfasst darüber hinaus ein Injektorrohr 3, welches in axialer Richtung nach hinten an die Injektorspitze 2 anschließt. Der Injektor 1 umfasst darüber hinaus einen Kolben 4, der in Richtung der Längsachse A in dem Injektorrohr 3 verschiebbar ist. In dem Injektorrohr 3 ist auch ein Ladebereich 5 ausgebildet, in dem eine Kassette 6 einsetzbar ist oder darin fest positioniert ist.

Die zu implantierende Intraokularlinse ist in der Kassette 6 aufgenommen. Die Kassette 6 ist in axialer Richtung und somit in Richtung der Längsachse A frontseitig und rückseitig offen, sodass die sich darin befindliche Intraokularlinse durch den Kolben 4 in Richtung der Injektorspitze 2 aus der Kassette 6 ausgeschoben werden kann und in die Injektorspitze 2 eingeschoben werden kann.

Die Kassette 6 kann ein für sich betrachtet eigenes, separates Bauteil sein. Sie kann jedoch auch einstückig mit der Injektorspitze 2 ausgebildet sein.

In Fig. 2 ist in einer perspektivischen Darstellung ein Ausführungsbeispiel einer Kassette 6 im geöffneten Zustand gezeigt. Die Kassette 6 umfasst ein erstes Kassettenteil 7, welches mit einem zweiten Kassettenteil 8 schwenkbar verbunden ist, wobei dazu das Verschwenken um eine Längsachse A der Kassette 6 ermöglicht ist. Die Kassettenteile 7 und 8 können somit in azimutaler Richtung aufeinander zu bewegt werden und voneinander weg bewegt werden. Die beiden Kassettenteile 7 und 8 sind über ein Filmscharnier 9 direkt miteinander verbunden. Die Kassette 6 ist einstückig aus Kunststoff ausgebildet.

Das Kassettenteil 7 umfasst ein Basiselement 10, welches rinnenförmig beziehungsweise bauchig ausgebildet ist. Das Kassettenteil 7 umfasst darüber hinaus einen plattenartigen Flügel 11, der mit dem Basiselement 10 verbunden ist und direkt an ein dem Filmscharnier 9 gegenüberliegendes Ende des Basiselements 10 anschließt. Darüber hinaus umfasst das weitere Kassettenteil 8 entsprechend ebenfalls ein Basiselement 12 und einen Flügel 13. Die Basiselemente 10 und 12 sind an den Flügeln 11 und 13 abgewandten gegenüberliegenden Enden mit dem Filmscharnier 9 direkt verbunden.

Die Kassette 6 umfasst darüber hinaus einen Aufnahmeraum beziehungsweise eine Aufnahmekammer 14, in welcher eine Intraokularlinse aufgenommen werden kann. Die Aufnahmekammer 14 ist einerseits durch die Innenseite der Basiselemente 10 und 12 begrenzt, andererseits durch einen Deckel 15 abgedeckt. Der Deckel 15 ist bei dieser Ausführungsform eine ebene, einteilige Platte, die die Aufnahmekammer 14 im geöffneten Zustand der Kassette 6 von oben verschließt. Der Deckel 15 ist an einem proximalen Ende mit einer Gelenkverbindung 16, insbesondere einem Filmscharnier, mit dem Kassettenteil 7 verbunden. Der Deckel 15 ist somit ebenfalls einstückig mit dem Kassettenteil 7 ausgebildet. Der Deckel 15 weist ein distales Ende 17 auf, welches auf einer, insbesondere planen, Innenseite des Kassettenteils 8, insbesondere einer Oberseite 30 des Flügels 13 aufliegt. Das distale Ende 17 ist daher hier nicht örtlich fest mit dem Kassettenteil 8 verbunden. Vielmehr gleitet beim Verschwenken der Kassettenteile 7 und 8 in eine geschlossene Stellung der Deckel 15 mit seinem distalen Ende 17 auf der Oberseite 30 entlang. Das distale Ende 17 kann abgerundet ausgebildet sein oder mit einer von der Oberseite 30 abgewandten Struktur bzw. Wölbung ausgebildet sein. Damit wird erreicht, dass während einer Schwenkbewegung des Flügels 11 und/oder des Flügels 13 ein Gleiten des Deckels 15 an der Oberseite 30 ohne Verkanten möglich ist. Die Gelenkverbindung 16 ist bevorzugt an einem Übergang zwischen dem Basiselement 10 und dem Flügel 11 vorgesehen.

Wie zu erkennen ist, umfasst sowohl das Kassettenteil 7 als auch das Kassettenteil 8 einen jeweiligen Steg 18 und 19. Die Stege 18 und 19 ragen radial in die Aufnahmekammer 14 hinein und liegen im geschlossenen Zustand der Kassette 6 direkt aneinander an. Durch diese radial orientierten Stege 18 und 19 wird auch ein in Umlaufrichtung um die Längsachse A vorgegebenes Faltanschlagelement gebildet, sodass sich hier der Faltvorgang der Intraokularlinse in der Kassette 6 beim Schließen nicht unerwünscht weit fortsetzen kann. Insbesondere ist die Verbindung des Deckels 15 mit dem Kassettenteil 7 beim Steg 18 ausgebildet.

An einer der Aufnahmekammer 14 abgewandten Außenseite 20 des Deckels 15 ist ein Stutzen 21 ausgebildet, der zylinderförmig ausgebildet ist und von der Außenseite 20 vorsteht. Dieser Stutzen 21 weist einen trichterförmigen Einlauf 22 auf. Ein Boden dieses trichterförmigen Einlaufs 22 mündet im Ausführungsbeispiel direkt in ein Einfüllloch 23, welches ein durch den Deckel 15 durchgängiges Loch ist, vgl. Fig. 2 und Fig. 12 bis 14. Durch dieses Einfüllloch 23 kann das Gleitmittel von außerhalb der Kassette 6 in die Aufnahmekammer 14 zugeführt werden. Durch den Stutzen 21 und insbesondere den trichterförmigen Einlauf 22 kann ein Hilfswerkzeug, in dem das Gleitmittel enthalten ist, einfach und zielsicher zum Einfüllloch 23 gebracht werden und beim Einfüllvorgang auch in seiner Position sicher gehalten werden.

Vorzugsweise weist der Einlauf 22 im Stutzen 21 an seiner schmalsten bzw. engsten Stelle einen Innendurchmesser auf, der kleiner ist als ein Außendurchmesser einer Injektionsnadel eines Hilfswerkzeugs zum Einfüllen des Gleitmittels. Insbesondere ist diese engste Stelle am Einlaufboden des Einlaufs 22. Ein Eintauchen der Injektionsnadel des Hilfswerkzeugs durch den Einlauf 22 und somit auch das Einfüllloch 23 hindurch bis zur Aufnahmekammer 14 ist dadurch verhindert. Zusätzlich oder anstatt dazu kann auch der Innendurchmesser des Einfüllochs 23 entsprechend klein dimensioniert sein.

Darüber hinaus ist vorgesehen, dass im Kassettenteil 7, welchem im geschlossenen Zustand der Kassette der Stutzen 21 zugewandt ist, eine Aussparung 24 ausgebildet ist, durch welche dann auch im geschlossenen Zustand der Kassette das Einfüllloch 23 zugänglich ist. Der Stutzen 21 erstreckt sich im geschlossenen Zustand der Kassette 6 in die Aussparung 24 zumindest hinein. An der Innenseite 50 des Deckels 15, welche der Außenseite 20 gegenüberliegt, kann eine rinnenartige Ausnehmung oder ein Zuführkanal vorgesehen sein, damit im geschlossenen Zustand der Kassette Gleitmittel vom Einfüllloch 23 entlang der rinnenartigen Ausnehmung zur Aufnahmekammer 14 gelangen kann.

Durch die gezeigte Kassette 6 kann somit sowohl im geöffneten Zustand der Kassette 6 als auch im geschlossenen Zustand der Kassette 6 eine Befüllung mit dem Gleitmittel vorgenommen werden.

Das Einfüllloch 23 ist mit seiner Lochachse insbesondere senkrecht zur planen Außenseite 20 des Deckels 15 orientiert.

Ausgehend von der in Fig. 2 gezeigten geöffneten Stellung der Kassette 6 kann diese in eine geschlossene Stellung gebracht werden, indem die beiden Kassettenteile 7 und 8 aufeinander zu bewegt werden, insbesondere die beiden Flügel 11 und 13 aufeinander zu bewegt werden und sich mittels einer durch die Flügel 11 und 13 gebildeten Schnappverbindung miteinander arretieren lassen. Bei diesem Schließvorgang wird eine Relativbewegung zwischen den Kassettenteilen 7 und 8 und dem Deckel 15 derart durchgeführt, dass der Deckel 15 mit seinem distalen Ende 17 auf der Oberseite 30 des Flügels 13 entlang gleitet, bis er im geschlossenen Zustand der Kassette 6 parallel als Zwischenlage beziehungsweise Zwischenplatte zwischen den Flügeln 11 und 13 angeordnet ist. Die Außenseite 20 des Deckels 15 liegt dann an einer Innenseite beziehungsweise Oberseite 25 des Flügels 11 an.

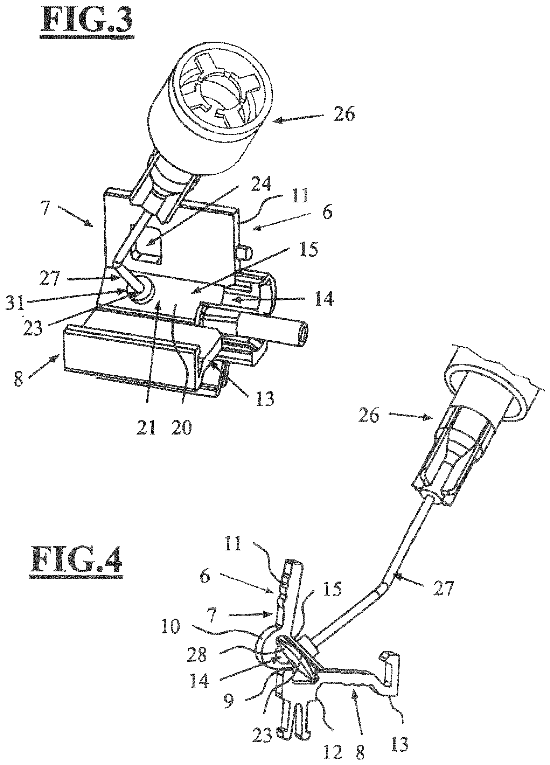

In Fig. 3 ist eine weitere perspektivische Darstellung der Kassette 6 gezeigt, wobei hier ein Hilfswerkzeug 26 mit einer Injektionsnadel 27 mit dem Einfüllloch 23 gekoppelt ist. Die Befüllung der Aufnahmekammer 14 ist hier im geöffneten Zustand der Kassette 6 vollzogen.

In Fig. 4 ist eine weitere perspektivische Darstellung der Ausgestaltung in Fig. 3 gezeigt. Die Intraokularlinse 28 ist hier in der Aufnahmekammer 14 enthalten, jedoch aufgrund des geöffneten Zustands der Kassette 6 noch nicht vorgefaltet. Sie ist daher in ihrem ungefalteten Zustand gezeigt. Es ist zu erkennen, dass die Einfüllöffnung beziehungsweise das Einfüllloch 23 auf der dem Filmscharnier 9 abgewandten Seite der Aufnahmekammer 14 angeordnet ist. Das Filmscharnier 9 und die Einfüllöffnung beziehungsweise das Einfüllloch 23 sind daher an einander gegenüberliegenden Seiten der Intraokularlinse 28 ausgebildet. Die Befüllung der Aufnahmekammer 14 mit dem Gleitmittel erfolgt somit auf einer dem Filmscharnier 9 abgewandten Seite. Dadurch wird die Intraokularlinse 28 durch das eingebrachte Gleitmittel gestützt. Das Vorfalten in die gewünschte Richtung, nämlich ausgehend von der in Fig. 4 gezeigten Grundstellung hin zum Filmscharnier 9, wird dadurch unterstützt.

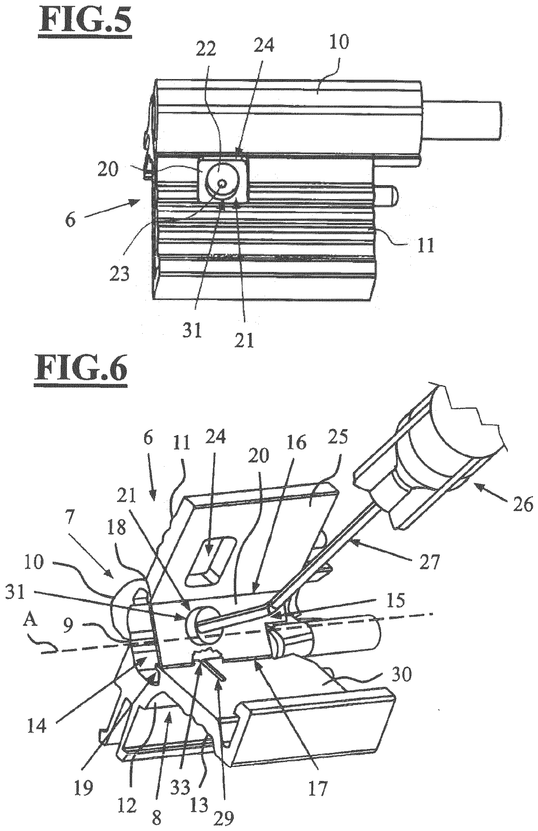

In Fig. 5 ist in einer perspektivischen Darstellung der geschlossene Zustand der Kassette 6 gezeigt. Es ist hier zu erkennen, dass das Einfüllloch 23 durch die Aussparung 24 in dem Flügel 11 zugänglich ist.

In Fig. 6 ist ein weiteres Ausführungsbeispiel einer Kassette 6 im geöffneten Zustand gezeigt. Bei dieser Ausführung ist im Unterschied zu den bisherigen Beispielen vorgesehen, dass ein zum Einfüllloch 23 separater und zusätzlicher rinnenartiger Zuführkanal 29 ausgebildet ist. Der rinnenartige Zuführkanal 29 ist nicht im Deckel 15, sondern in einem Kassettenteil, hier im Kassettenteil 8, ausgebildet. Insbesondere ist er in dem Flügel 13 des Kassettenteils 8 ausgebildet. Er ist an der Oberseite 30 des Flügels 13 ausgebildet und nach oben hin und somit auf der dem Kassettenteil 7 zugewandten Seite radial (zu seiner Längserstreckung betrachtet) offen gestaltet. Im geschlossenen Zustand der Kassette 6 ist er somit zum anderen Kassettenteil 7 parallel verlaufend und diesem zugewandt und offen ausgebildet. Der rinnenartiger Zuführkanal 29 ist entlang seiner Längsachse bzw. Kanalachse betrachtet auf der der Aufnahmekammer 14 abgewandten Seite geschlossen, auf der der Aufnahmekammer 14 zugewandten Seite offen und mündet in die Aufnahmekammer 14. Er verläuft somit über den Steg 19 bzw. das Podest zur Aufnahmekammer 14. Im geschlossenen Zustand der Kassette 6 kann somit auch bei dieser Positionierung des Stutzens 21 und des Einfülllochs 23 eine Befüllung erfolgen. Obwohl dann der Deckel 15 parallel zwischen dem Flügel 11 und dem Flügel 13 angeordnet ist, kann das Gleitmittel durch das Einfüllloch 23 direkt zu dem dann örtlich dazu korrespondierenden und lagegenau angeordneten rinnenartigen Zuführkanal 29 von außen zur Aufnahmekammer 14 geleitet werden. Im geschlossenen Zustand der Kassette 6 mündet das Einfüllloch 23 daher direkt in den rinnenartigen Zuführkanal 29. Bevorzugt ist eine zur Längsachse A senkrechte Einmündung des rinnenartigen Zuführkanals 29 in die Aufnahmekammer 14 ausgebildet und ein Kanalende 33 ausgebildet, das in dem Steg 19 ausgebildet ist. Zum Erkennen des Kanalendes 33 ist der Deckel 15 in Fig. 6 aufgebrochen gezeigt.

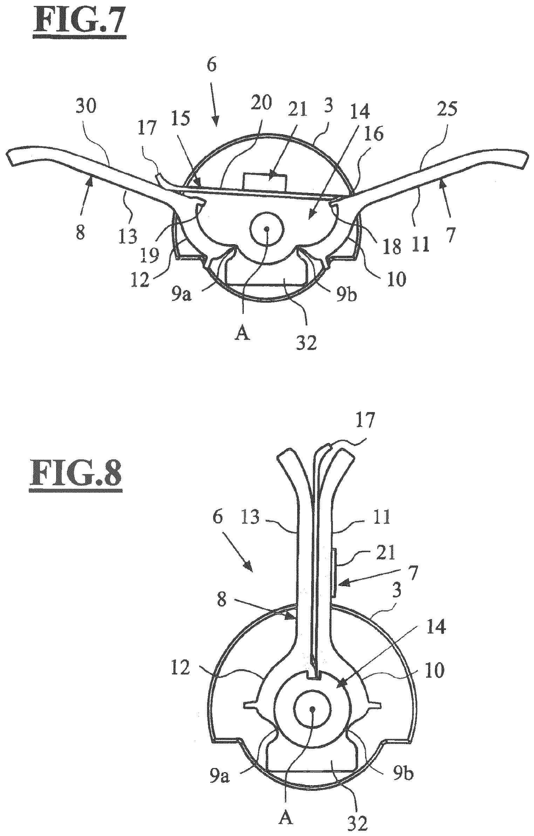

In Fig. 7 ist in einer Seitenansicht ein weiteres und bevorzugtes Ausführungsbeispiel einer erfindungsgemäßen Kassette 6 im geöffneten Zustand gezeigt. Die Kassette 6 ist in dem Injektorrohr 3 angeordnet dargestellt. Bei diesem Beispiel ist zusätzlich zu den Kassettenteilen 7 und 8 zumindest ein Sockelelement 32 ausgebildet. Das Sockelelement 32 begrenzt mit einer Innenseite die Aufnahmekammer 14. Es ist mit einer ersten Gelenkverbindung 9a, insbesondere einem Filmschamier, mit dem Basiselement 12 und mit einer zweiten Gelenkverbindung 9b, insbesondere einem Filmscharnier, mit dem Basiselement 10 gelenkig verbunden.

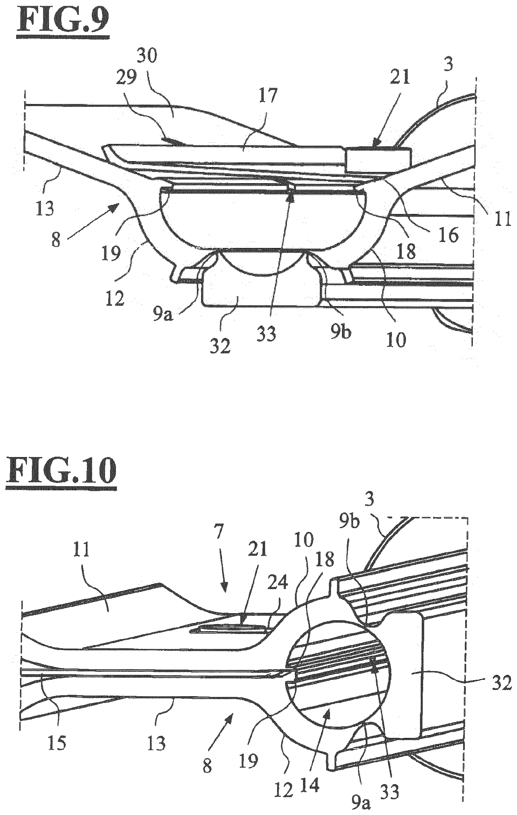

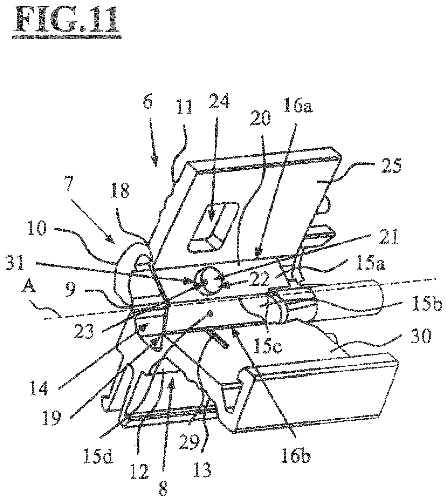

In Fig. 8 ist die Kassette 6 gemäß Fig. 7 im geschlossenen Zustand gezeigt. Der Stutzen 21 ragt durch die Aussparung 24 hindurch. Es kann der rinnenartige Zuführkanal 29 vorgesehen sein, wie dies in den weiteren Ausführungen gemäß Fig. 9 und 10 gezeigt ist. Bei der in Fig. 11 dargestellten Ausführungsform ist im Unterschied zur Ausgestaltung in Fig. 2 vorgesehen, dass der Deckel 15 mehrteilig ausgebildet ist und im Beispiel zumindest zwei Plattenteile 15a und 15b aufweist, die mit einem Filmscharnier 15c als Gelenkverbindung gelenkig miteinander verbunden sind. Der Deckel 15 ist hier gelenkig mit beiden Kassettenteilen 7 und 8 verbunden, wobei insbesondere Filmscharniere 16a und 16b ausgebildet sind. Der Deckel 15 ist somit ebenfalls einstückig mit den Kassettenteilen 7 und 8 ausgebildet. Die länglichen Plattenteile 15a und 15b sind somit an ihren gegenüberliegenden parallelen, und parallel zur Längsachse A verlaufenden Längsseiten örtlich fest mit den jeweiligen Kassettenteilen 7 und 8 jedoch relativ beweglich zu den Kassettenteilen 7 und 8 verbunden. Die Verbindungsstellen sind hier definiert so gewählt, dass sie jeweils an innenseitigen Übergängen zwischen einem Basiselement 10, 12 und einem Flügel 11, 13 der Kassettenteile 7 und 8 angeordnet sind. Der Abstand zwischen dem Filmscharnier 16a zu dem Filmscharnier 15c einerseits und dem Filmscharnier 16b zu dem Filmscharnier 15c andererseits ist vorzugsweise gleich groß, so dass im geschlossenen Zustand der Kassette 6 die Plattenteile 15a und 15b im Wesentlichen deckungsgleich aufeinander liegen. Insbesondere ist der Stutzen 21 nur an dem Plattenteil 15a ausgebildet. Das Gleitmittel kann dann durch den Stutzen 21 im geöffneten Zustand der Kassette 6 in die Aufnahmekammer 14 gebracht werden.

Soll die Kassette 6 auch im geschlossenen Zustand mit Gleitmittel in der Aufnahmekammer 14 befüllbar sein, so ist bei einer derartigen Ausführung dann in einem weiteren Ausführungsbeispiel an dem Plattenteil 15b ein Durchgangsloch 15d als Einfüllloch ausgebildet, welches im geschlossenen Zustand der Kassette 6 fluchtend mit dem Einfüllloch 23 angeordnet ist.

Es ist bei dieser Ausführung auch ein rinnenartiger Zuführkanal 29 ausgebildet, welcher in Fig. 11 der Übersichtlichkeit dienend nur für ein Ausführungsbeispiel in der Oberseite 30 gezeigt ist. Dieser kann in der Oberseite 30 ausgebildet sein und wie bei den bisherigen Ausführungen geradlinig oder nicht-geradlinig ausgebildet sein. Dieser rinnenartige Zuführkanal 29 durchstößt bei dieser Ausführungsform an seinem Kanalende auch das Filmscharnier 16b.

Der rinnenartige Zuführkanal 29 kann bei einer alternativen Ausführung auch in einer der Aufnahmekammer 14 zugewandten Innenseite des Plattenteils 15a und/oder in einer der Aufnahmekammer 14 zugewandten Innenseite des Plattenteils 15b ausgebildet sein. An der Innenseite des Plattenteils 15a mündet er dann direkt an das Einfüllloch 23 in dem Plattenteil 15a, an welches der Einlauf 22 des Stutzens 21 auch mündet. Ist er an der Innenseite des Plattenteils 15b ausgebildet, so mündet er erst im geschlossenen Zustand der Kassette 6 an das Einfüllloch 23 in dem Plattenteil 15a. Ist der Zuführkanal 29 in beiden Innenseiten ausgebildet, ist er im geschlossenen Zustand der Kassette 6 nicht nur in Umlaufrichtung um die Kanalachse als eine Halbrinne ausgebildet, sondern als aus zwei Halbrinnen gebildeter Kanal ausgebildet. Bei diesen Ausführungen mit dem Zuführkanal 29 in der Innenseite des Plattenteils 15a und/oder des Plattenteils 15b ist das Durchgangsloch 15d dann nicht vorhanden.

Die Ausführungsbeispiele gemäß Fig. 11 können auch mit zumindest einem Sockelelement 32 ausgebildet sein, wie dies bei den Ausführungsformen in Fig. 7 bis 10 vorhanden ist.

Bei weiteren Ausführungsformen gemäß Fig. 11 kann auch vorgesehen sein, dass der Einlauf 22 sehr niedrig ausgebildet ist, wobei das Einfüllloch 23 und die Aussparung 24 vorhanden sind. Vorzugsweise kann dann das Einfüllloch 23 in Verbindung mit dem Durchgangsloch 15d im zusammengeklappten Zustand der Kassette 6 eine Führung bzw. Aufnahme für die Injektionsnadel 27 bilden, insbesondere derart, dass die Injektionsnadel 27 nicht in die Aufnahmekammer 14 eindringen kann. Insbesondere können dann auch mehr als zwei Plattenteile 15a und 15b vorhanden sein, die ziehharmonikaartig sich aufeinander legen können, wenn die Kassette 6 geschlossen wird, so dass ein mehrlagiger Bereich zwischen den Flügeln 11 und 13 erzeugt ist, der durch seine Dicke dann das Halten und Führen der Injektionsnadel 27 begünstigt.

Die Kassette 6 weist bei den von der Erfindung umfassten Ausführungen eine Gleitmittel-Zuführvorrichtung 31 auf, die das Einfüllloch 23 und insbesondere auch den rinnenartigen Zuführkanal 29 und insbesondere den Stutzen 21 und insbesondere die Aussparung 24 aufweist.

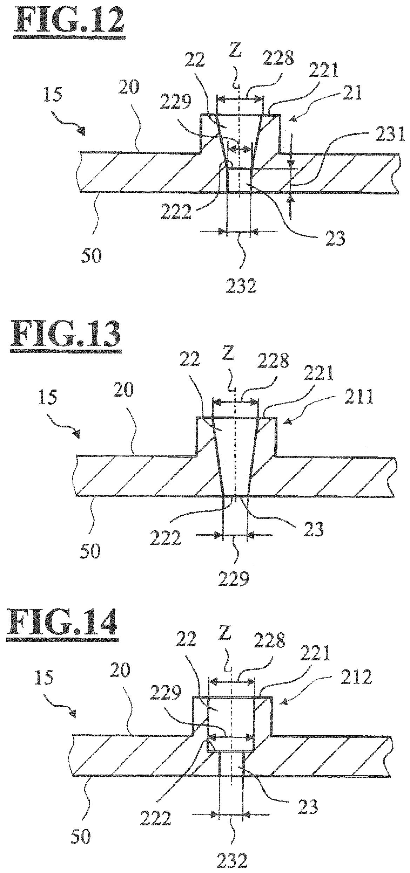

Die Figuren 12 bis 14 zeigen Querschnittsansichten von Stutzen, die von der Außenseite 20 des Deckels 15 vorstehen. Der erste Stutzen 21 gemäß Fig. 12 weist einen Einlauf 22 auf, der trichterförmig um eine Achse Z ausgebildet ist, wobei die Achse Z senkrecht zu einer Ebene verläuft, die durch die Außenseite 20 gebildet ist. Der Einlauf 22 weist einen Durchmesser 228 an einem proximalen Ende 221 auf, welches von der Außenseite 20 maximal beabstandet ist, wobei der Durchmesser 228 größer ist als der Durchmesser 229 an einem distalen Ende 222 des Einlaufs 22. Der trichterförmige Einlauf 22 verjüngt sich somit von dem proximalen Ende 221 in Richtung zum distalen Ende 222. Der Einlauf 22 mündet mit seinem distalen Ende 222 in das Einfüllloch 23, wobei der Durchmesser am distalen Ende 222 gleich einem Durchmesser 232 des Einfüllloches 23 ist. Das Einfüllloch 23 ist als Durchgangsloch mit konstantem Durchmesser entlang einer Höhe 231 ausgebildet. Der Durchmesser 232 muss derart dimensioniert sein, dass er kleiner als der größte Durchmesser einer in den Einlauf 22 einzuführenden Injektionsnadel 27 ist, sodass die Injektionsnadel 27 nicht das Einfüllloch 23 erreichen und eine eventuell darunter angeordnete Intraokularlinse 28 beschädigen kann.

Fig. 13 zeigt eine Querschnittsansicht eines zweiten Stutzens 211, der von der Außenseite 20 des Deckels 15 vorsteht. Der Stutzen 211 weist ein proximales Ende 221 auf, das von der Außenseite 20 des Deckels maximal beabstandet ist und eine von außen zugängliche Öffnung des Einlaufes 22 bildet. Der Einlauf 22 ist trichterförmig um eine Achse Z ausgebildet, welche senkrecht zu einer Ebene verläuft, die durch die Außenseite 20 des Deckels 15 gebildet ist. Der Einlauf 22 verjüngt sich vom proximalen Ende 221 in Richtung zu einem distalen Ende 222 zunehmend. Der Durchmesser 228 am proximalen Ende 221 des Einlaufs 22 ist somit größer als der Durchmesser 229 am distalen Ende des Einlaufs 22. Das distale Ende 222 verläuft bündig mit der Innenseite 50 des Deckels 15, sodass das distale Ende 222 des Einlaufs 22 das Einfüllloch 23 bildet.

Fig. 14 zeigt eine Querschnittsansicht eines dritten Stutzens 212, der ähnlich zu dem ersten Stutzen 21 ist. Der dritte Stutzen 212 steht von der Außenseite 20 des Deckels 15 vor und weist ein proximales Ende 221 auf, welches von der Außenseite 20 maximal beabstandet ist. Der Einlauf 22 beginnt am proximalen Ende 221 des Stutzens 212 und reicht bis zu einem distalen Ende 222, wobei der Einlauf zylinderförmig um eine Achse Z ausgebildet ist. Der Einlauf 22 bildet bei diesem dritten Stutzen 212 ein Sackloch. Der Einlauf 22 mündet am distalen Ende 222 in das Einfüllloch 23, wobei der Durchmesser 229 des Einlaufs 22 größer ist als der Durchmesser 232 des Einfüllloches 23. Diese Ausführungsform hat den Vorteil, dass eine Injektionsnadel 27 auf das distale Ende 222 des Einlaufs 22 aufgesetzt werden kann und damit sicher im Einlauf 22 platzierbar ist.

## Patentansprüche

1. Kassette (6) zur Aufnahme einer Intraokularlinse (28) für einen Injektor (1) zum Einführen der Intraokularlinse (28) in ein Auge, mit zwei um eine Längsachse (A) der Kassette (6) relativ zueinander schwenkbaren Kassettenteilen (7, 8), wobei ein Kassettenteil (7) ein rinnenartiges Basiselement (10) und einen daran anschließenden plattenartigen Flügel (11) aufweist, wobei dieses Kassettenteil (7) mit einem Deckel (15) gelenkig verbunden ist, und ein weiteres Kassettenteil (8) ein rinnenartiges Basiselement (12) und einen daran anschließenden plattenartigen Flügel (13) aufweist, und im geschlossenen Zustand der Kassette (6) durch die beiden Basiselemente (10, 12) zumindest ein Teil einer Aufnahmekammer (14) für die Intraokularlinse (28) gebildet ist, und mit einer Gleitmittel-Zuführeinrichtung (31), mit welcher ein Gleitmittel für die Intraokularlinse (28) in die Aufnahmekammer (14) zuführbar ist, wobei der Deckel (15) zum Abdecken der Aufnahmekammer (14) vorgesehen ist, und ein Einfüllloch (23) der Gleitmittel-Zuführeinrichtung (31) in dem Deckel (15) ausgebildet ist,
**dadurch gekennzeichnet, dass**
an einer der Aufnahmekammer (14) abgewandten Außenseite (20) des Deckels (15) ein Stutzen (21; 211; 212) vorsteht, der einen Einlauf (22) aufweist, der in das Einfüllloch (23) mündet und in dem Flügel (13) des weiteren Kassettenteils (8) oder in dem Deckel (15) ein rinnenartiger Zuführkanal (29) der Gleitmittel-Zuführeinrichtung (31) ausgebildet ist, der in die Aufnahmekammer (14) mündet und im geschlossenen Zustand der Kassette (6) mit dem Einfüllloch (23) zur Einleitung von Gleitmittel vom Einfüllloch (23) zur Aufnahmekammer (14) zusammenwirkt.

2. Kassette (6) nach Anspruch 1,
**dadurch gekennzeichnet, dass**
der Einlauf (22) trichterförmig ausgebildet ist.

3. Kassette (6) nach einem der vorhergehenden Ansprüche,
**dadurch gekennzeichnet, dass**
ein Kassettenteil (7, 8) in seinem Flügel (11, 13) eine Aussparung (24) aufweist, durch welche der Stutzen (21; 211; 212) im geschlossenen Zustand der Kassette (6) von außen zugänglich ist.

4. Kassette (6) nach Anspruch 3,
**dadurch gekennzeichnet, dass**
sich der Stutzen (21; 211; 212) im geschlossenen Zustand der Kassette (6) in die Aussparung (24) hinein erstreckt.

5. Kassette (6) nach einem der vorhergehenden Ansprüche,
**dadurch gekennzeichnet, dass**
der rinnenartige Zuführkanal (29) im geschlossenen Zustand der Kassette (6) senkrecht zu einer Lochachse des Einfülllochs (23) verlaufend angeordnet ist und das Einfüllloch (23) an dem Zuführkanal (29) mündet.

6. Kassette (6) nach einem der vorhergehenden Ansprüche,
**dadurch gekennzeichnet, dass**
der rinnenartige Zuführkanal (29) in dem Flügel (11, 13) des Kassettenteils (7, 8) ausgebildet ist oder an einer der Aufnahmekammer (14) zugewandten Innenseite (50) des Deckels (15) ausgebildet ist.

7. Kassette (6) nach einem der vorhergehenden Ansprüche,
**dadurch gekennzeichnet, dass**
der Deckel (15) als einteilige Platte ausgebildet ist, und der Deckel (15) an nur einer Längsseite mit einem Filmscharnier (16) mit einem Kassettenteil (7) verbunden ist und an der anderen Längsseite auf dem anderen Kassettenteil (8) verschiebbar aufliegend angeordnet ist.

8. Kassette (6) nach einem der vorhergehenden Ansprüche,
**dadurch gekennzeichnet, dass**
der Deckel (15) mit einem ersten Plattenteil (15a), an dem der Stutzen (21; 211; 212) ausgebildet ist, und mit zumindest einem zweiten Plattenteil (15b) ausgebildet ist, wobei die Plattenteile (15a, 15b) mit einem Filmscharnier (15c) schwenkbar direkt miteinander verbunden sind, und das erste Plattenteil (15a) mit einem Filmscharnier (16a) mit dem ersten Kassettenteil (7) und das zweite Plattenteil (15b) mit einem Filmscharnier (16b) mit dem zweiten Kassettenteil (8) verbunden ist.

9. Injektor (1) zum Einführen einer Intraokularlinse (28) in ein Auge, welcher eine Kassette (6) nach einem der vorherigen Ansprüche aufweist.

## Claims

1. Cartridge (6) for receiving an intraocular lens (28) for an injector (1) for introducing the intraocular lens (28) into an eye, having two cartridge parts (7, 8) that are pivotable relative to one another about a longitudinal axis (A) of the cartridge (6), wherein one cartridge part (7) has a channel-like base element (10) and a plate-like wing (11) adjoining the latter, wherein said cartridge part (7) is connected in an articulated manner to a cover (15), and a further cartridge part (8) has a channel-like base element (12) and a plate-like wing (13) adjoining the latter, and, in the closed state of the cartridge (6), at least a part of a receiving chamber (14) for the intraocular lens (28) is formed by the two base elements (10, 12), and having a lubricant feed device (31) with which a lubricant for the intraocular lens (28) is feedable into the receiving chamber (14), wherein the cover (15) is provided for covering the receiving chamber (14), and a filling hole (23) of the lubricant feed device (31) is formed in the cover (15),
**characterized in that**
a nozzle (21; 211; 212) protrudes on an outer side (20) of the cover (15) that faces away from the receiving chamber (14), the nozzle having an inlet (22) which opens into the filling hole (23), and a channel-like feed duct (29) of the lubricant feed device (31) is formed in the wing (13) of the further cartridge part (8) or in the cover (15), said channel-like feed duct (29) opening into the receiving chamber (14) and, in the closed state of the cartridge (6), interacting with the filling hole (23) for conducting lubricant from the filling hole (23) to the receiving chamber (14).

2. Cartridge (6) according to Claim 1,
**characterized in that**
the inlet (22) is formed in a funnel-shaped manner.

3. Cartridge (6) according to either of the preceding claims,
**characterized in that**
the wing (11, 13) of one cartridge part (7, 8) has a cutout (24) through which the nozzle (21; 211; 212) is accessible from the outside in the closed state of the cartridge (6).

4. Cartridge (6) according to Claim 3,
**characterized in that**
the nozzle (21; 211; 212) extends into the cutout (24) in the closed state of the cartridge (6).

5. Cartridge (6) according to one of the preceding claims,
**characterized in that**,
in the closed state of the cartridge (6), the channel-like feed duct (29) is arranged running perpendicularly to a hole axis of the filling hole (23) and the filing hole (23) opens at the feed duct (29).

6. Cartridge (6) according to one of the preceding claims,
**characterized in that**
the channel-like feed duct (29) is formed in the wing (11, 13) of the cartridge part (7, 8) or is formed on an inner side (50) of the cover (15) that faces the receiving chamber (14).

7. Cartridge (6) according to one of the preceding claims,
**characterized in that**
the cover (15) is in the form of a single-part plate, and the cover (15) is connected on only one longitudinal side to one cartridge part (7) by a film hinge (16) and is arranged on the other longitudinal side in a manner resting displaceable on the other cartridge part (8).

8. Cartridge (6) according to one of the preceding claims,
**characterized in that**
the cover (15) is formed with a first plate part (15a) on which the nozzle (21; 211; 212) is formed, and with at least one second plate part (15b), wherein the plate parts (15a, 15b) are connected directly to one another in a pivotable manner by a film hinge (15c), and the first plate part (15a) is connected to the first cartridge part (7) by a film hinge (16a) and the second plate part (15b) is connected to the second cartridge part (8) by a film hinge (16b).

9. Injector (1) for introducing an intraocular lens (28) into an eye, said injector (1) having a cartridge (6) according to one of the preceding claims.

## Revendications

1. Cassette (6) destinée à loger une lentille intraoculaire (28) pour un injecteur (1) servant à insérer la lentille intraoculaire (28) dans un œil, comprenant deux parties de cassette (7, 8) pouvant pivoter l'une par rapport à l'autre autour d'un axe longitudinal (A) de la cassette (6), une partie de cassette (7) comprenant un élément de base (10) en forme de rigole et une ailette (11) en forme de plaque, adjacente à ce dernier, cette partie de cassette (7) étant reliée de manière articulée à un couvercle (15), et une partie de cassette supplémentaire (8) comprenant un élément de base (12) en forme de rigole et une ailette (13) en forme de plaque, adjacente à ce dernier, et, dans l'état fermé de la cassette (6), au moins une partie d'une chambre de logement (14) pour la lentille intraoculaire (28) étant formée par les deux éléments de base (10, 12), et comprenant un dispositif d'alimentation en lubrifiant (31), avec lequel un lubrifiant pour la lentille intraoculaire (28) peut être acheminé dans la chambre de logement (14), le couvercle (15) étant prévu pour recouvrir la chambre de logement (14), et un orifice de remplissage (23) du dispositif d'alimentation en lubrifiant (31) étant formé dans le couvercle (15),
**caractérisée en ce que**
un embout (21 ; 211 ; 212), qui comprend une entrée (22) qui débouche dans l'orifice de remplissage (23), fait saillie sur un côté extérieur (20), détourné de la chambre de logement (14), du couvercle (15), et un canal d'alimentation (29) en forme de rigole du dispositif d'alimentation en lubrifiant (31), qui débouche dans la chambre de logement (14), est formé dans l'ailette (13) de la partie de cassette supplémentaire (8) ou dans le couvercle (15), et coopère dans l'état fermé de la cassette (6) avec l'orifice de remplissage (23) pour l'introduction de lubrifiant depuis l'orifice de remplissage (23) dans la chambre de logement (14).

2. Cassette (6) selon la revendication 1, **caractérisée en ce que**
l'entrée (22) est configurée en forme d'entonnoir.

3. Cassette (6) selon l'une quelconque des revendications précédentes,
**caractérisée en ce que**
une partie de cassette (7, 8) comprend dans son ailette (11, 13) un évidement (24), à travers lequel l'embout (21 ; 211 ; 212) est accessible depuis l'extérieur dans l'état fermé de la cassette (6).

4. Cassette (6) selon la revendication 3, **caractérisée en ce que**
l'embout (21 ; 211 ; 212) s'étend dans l'évidement (24) dans l'état fermé de la cassette (6).

5. Cassette (6) selon l'une quelconque des revendications précédentes,
**caractérisée en ce que**
le canal d'alimentation (29) en forme de rigole est agencé perpendiculairement à un axe d'orifice de l'orifice de remplissage (23) dans l'état fermé de la cassette (6), et l'orifice de remplissage (23) débouche au niveau du canal d'alimentation (29).

6. Cassette (6) selon l'une quelconque des revendications précédentes,
**caractérisée en ce que**
le canal d'alimentation (29) en forme de rigole est formé dans l'ailette (11, 13) de la partie de cassette (7, 8) ou est formé sur un côté intérieur (50) du couvercle (15) tourné vers la chambre de logement (14).

7. Cassette (6) selon l'une quelconque des revendications précédentes,
**caractérisée en ce que**
le couvercle (15) est configuré en tant que plaque d'un seul tenant, et le couvercle (15) est relié à une partie de cassette (7) uniquement au niveau d'un côté longitudinal avec une charnière pelliculaire (16), et repose de manière coulissante sur l'autre partie de cassette (8) au niveau de l'autre côté longitudinal.

8. Cassette (6) selon l'une quelconque des revendications précédentes, **caractérisée en ce que** le couvercle (15) est configuré avec une première partie de plaque (15a), sur laquelle l'embout (21 ; 211 ; 212) est formé, et avec au moins une deuxième partie de plaque (15b), les parties de plaque (15a, 15b) étant reliées directement l'une à l'autre de manière à pouvoir pivoter avec une charnière pelliculaire (15c), et la première partie de plaque (15a) étant reliée à la première partie de cassette (7) avec une charnière pelliculaire (16a) et la deuxième partie de plaque (15b) étant reliée à la deuxième partie de cassette (8) avec une charnière pelliculaire (16b).

9. Injecteur (1) servant à insérer une lentille intraoculaire (28) dans un œil, qui comprend une cassette (6) selon l'une quelconque des revendications précédentes.
